# EUROPEAN PATENT APPLICATION

(11) **EP 4 692 369 A1**
(43) Date of publication of application: **11.02.2026**
(21) Application number: 25193359.4
(22) Date of filing: 01.08.2025
(51) Int. Cl.: C12Q 1/6834, C12Q 1/6837

(54) **METHOD FOR VIRUS DETECTION**

(30) Priority: 09.08.2024 TW 113129976
(71) Applicant: Locus Cell Co., Ltd., 221 New Taipei City (TW)
(72) Inventor: Liu, Heng-Yu, New Taipei City (TW); Chen, Hui-Ling, New Taipei City (TW); Chen, Szu-Yu, New Taipei City (TW); Lu, Tsung-Chi, New Taipei City (TW)
(74) Representative: Wang, Bo

(57) **Abstract**

A method for virus detection is applied to detect a plurality of viruses at one time and the method comprises the following steps of: coating a nucleic acid primer sequence corresponding to each of the viruses on a solid-phase carrier, wherein the viruses originate from different species; inputting a sample into the solid-phase carrier; setting a plurality of amplification reaction conditions, wherein the amplification reaction conditions comprise an amplification reaction temperature, an amplification primer length, an amplification primer concentration and a number of amplification reaction cycles; and conducting an amplification reaction on the sample input into the solid-phase carrier according to the amplification reaction conditions, so as to detect the plurality of viruses originating from different species at one time under the amplification reaction.

## Description

### BACKGROUND OF THE INVENTION

### 1. Field of the invention

The present invention relates to a method for virus detection, and more particularly, to a method for virus detection performed on cultured cells in the field of cell therapy.

### 2. Description of the prior art

Because of the specific nature of the cell therapy products, whose principal component is living cell, terminal sterilization cannot be applied and purification, viral clearance and viral inactivation steps cannot be performed at a large-scale during the manufacturing process. Therefore, the source of raw materials used in the manufacture of cell therapy products, especially the source of primary cells and any materials or the reagents of animal origin, must be strictly controlled. Users are required to describe in detail the sources of materials utilized in the manufacturing process and to list the test items applied to these materials to ensure the product safety and quality.

The cell source and the quality control are critical steps in the manufacturing process of the cell therapy product. The source of cells comprising collection methods, donor screening and cell-database system needs to be managed in a variety of ways. Otherwise, donors of same-type allogeneic cells should undergo screening and testing for specific infectious agents, such as Human Immunodeficiency Virus and Hepatitis A Virus. Simultaneously, cultured cells must be tested for relevant viruses tests when reagents derived from animal source, such as bovine serum or porcine trypsin.

In response to the increasing demand for the cell therapy, finding rapid and accurate methods for virus detection has become crucial. Current methods for virus detection typically target only a single virus or a limited number of viruses from the same species, which restricts their applicability to viruses or infection from different species. Besides, the multiplex PCR technology has been applied on detecting the plurality of viruses simultaneously. This technology can amplify a plurality of target nucleic acids by designing a plurality of specific amplification primers. However, the current multiplex PCR technology encounters several challenges, such as nonspecific binding between primers, variability in amplification efficiency and suboptimal reaction conditions.

Therefore, there is an urgent need to develop a novel method for virus detection which can efficiently and accurately detect the plurality of viruses originating from different species under one reaction to increase the diagnose efficiency and provide reliable detection results.

### SUMMARY OF THE INVENTION

Therefore, the present invention provides a method for virus detection to solve the problems of the prior art.

The present invention provides the method for virus detection, applied to detect a plurality of viruses at one time, and the method comprises the following steps of: coating a nucleic acid primer sequence corresponding to each of the viruses on a solid-phase carrier, wherein the viruses originate from different species; inputting a sample into the solid-phase carrier; setting a plurality of amplification reaction conditions, wherein the amplification reaction conditions comprise an amplification reaction temperature, an amplification primer length, an amplification primer concentration and a number of amplification reaction cycles; and conducting an amplification reaction on the sample input into the solid-phase carrier according to the amplification reaction conditions, so as to detect the plurality of viruses originating from different species at one time under the amplification reaction.

Wherein, the amplification reaction temperature is below 70°C, the amplification primer length is between 14 mer and 30 mer, the amplification primer concentration is between 0.1µM and 1µM, and the number of amplification reaction cycles is less than 40.

Wherein, the step of setting the plurality of amplification reaction conditions further comprises the following steps of: confirming an amplification reaction condition with the highest sensitivity; optimizing the other amplification reaction conditions under the fixed amplification reaction condition with the highest sensitivity; and setting a fixed optimal condition from the amplification reaction condition with the highest sensitivity and the other amplification reaction conditions after optimization; wherein, the step of conducting the amplification reaction on the sample input into the solid-phase carrier according to the amplification reaction conditions is conducting the amplification reaction by setting the fixed optimal condition.

Wherein, the step of setting the plurality of amplification reaction conditions further comprises the following steps of: optimizing the amplification reaction condition with the highest sensitivity under the other expansion reaction conditions that have been fixed and optimized; continuing to optimize the other amplification reaction conditions under the amplification reaction condition with the highest sensitivity that has been fixed and optimized; and setting the fixed optimal condition from the amplification reaction condition with the highest sensitivity after optimization, and from the other amplification reaction conditions after continuous optimization.

Wherein, the method for virus detection further comprises a nucleic acid purification reaction, a reverse transcription reaction, a polymerase chain reaction, and an amplification reaction analysis.

Wherein, the viruses comprise a human RNA virus, a human DNA virus, a bovine DNA virus and a porcine DNA virus.

Wherein, the human RNA virus comprises a Human Immunodeficiency Virus Type 1 (HIV-I), a Human Immunodeficiency Virus Type 2 (HIV-II), a Hepatitis A Virus (HAV), a Hepatitis C Virus (HCV), a Human T-Lymphotropic Virus Type 1 (HTLV-I), and a Human T-Lymphotropic Virus Type 2 (HTLV-II).

Wherein, the human DNA virus comprises a Hepatitis B Virus (HBV),a Parcovirus B19, an Epstein-Barr virus (EBV), a Human Cytomegalovirus (HCMV), a Human Adenovirus (HAdV),a Human Herpesvirus 6 (HHV-6), a Human Herpesvirus 7 (HHV-7),a Human Herpesvirus 8 (HHV-8), a Human Papillomavirus Type 16 (HPV-16), a Human Papillomavirus Type 18 (HPV-18), a BK virus and a JC virus.

Wherein, the bovine DNA virus comprises a Bovine Polyomavirus (BPyV).

Wherein, the porcine DNA virus comprises a Porcine Circovirus Type 1 (PCV-1), a Porcine Circovirus Type 2 (PCV-2) and a Porcine parvovirus (PPV).

In summary, the present invention provides the method for virus detection. Compared to the conventional technology which only detects the virus originating from the same species, the invention can detect the plurality of viruses originating from different species at one time by coating a nucleic acid primer sequence corresponding to each of the viruses on a solid-phase carrier, and adjusting and designing an amplification reaction condition. In addition, compared to the conventional technology that require costly and inefficient trial-and-error testing to adjust reaction conditions, the invention replaces the traditional trial-and-error test by identifying an amplification reaction condition with the highest sensitivity, thereby reducing cost and improving efficiency for virus detection. Moreover, compared to the insufficient precision of the conventional technology, the invention further increases the accuracy of the virus detection and reduces the incidence of false-negative or false-positive outcomes in viral diagnostics by optimizing the amplification reaction condition with the highest sensitivity and continuously optimizing the other amplification reaction conditions.

### BRIEF DESCRIPTION OF THE APPENDED DRAWINGS

FIG. 1 is a flowchart illustrating the steps of the method for virus detection according to an embodiment of the present invention.
FIG. 2 is a flowchart illustrating the steps of the method for virus detection according to an embodiment of the present invention.
FIG. 3 is a flowchart illustrating the steps of the method for virus detection according to an embodiment of the present invention.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion. The step numbers in this invention are provided solely to distinguish between different steps and do not indicate the order in which the steps are to be performed.

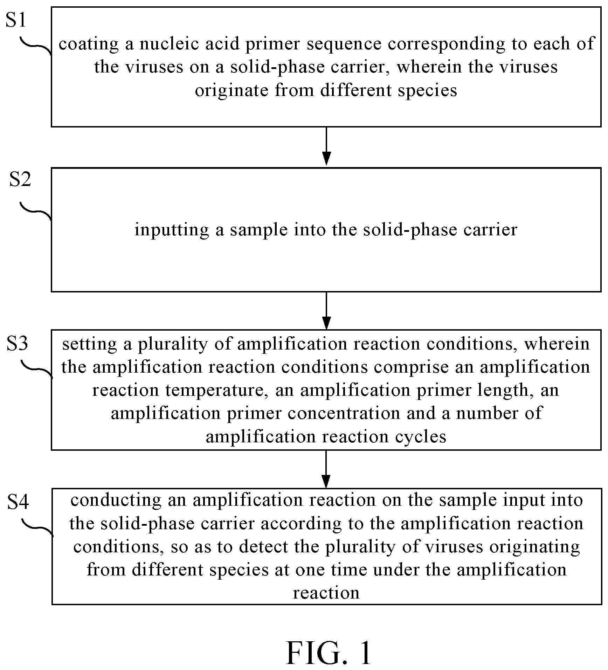

Please refer to FIG. 1. FIG. 1 is a flowchart illustrating the steps of the method for virus detection according to an embodiment of the present invention. As shown in FIG. 1. In this specific embodiment, the method for virus detection is applied to detect a plurality of viruses at one time. In this specific embodiment, the method for virus detection comprises the following steps of: Step S1: coating a nucleic acid primer sequence corresponding to each of the viruses on a solid-phase carrier, wherein the viruses originate from different species; Step S2: inputting a sample into the solid-phase carrier; Step S3: setting a plurality of amplification reaction conditions, wherein the amplification reaction conditions comprise an amplification reaction temperature, an amplification primer length, an amplification primer concentration and a number of amplification reaction cycles; and Step S4: conducting an amplification reaction on the sample input into the solid-phase carrier according to the amplification reaction conditions, so as to detect the plurality of viruses originating from different species at one time under the amplification reaction. In this specific embodiment, the method for virus detection further comprises a nucleic acid purification reaction, a reverse transcription reaction, a polymerase chain reaction, and an amplification reaction analysis.

In addition, in this specific embodiment, the viruses detected by the method for virus detection comprise a human RNA virus, a human DNA virus, a bovine DNA virus and a porcine DNA virus. Wherein, the human RNA virus comprises a Human Immunodeficiency Virus Type 1 (HIV-I), a Human Immunodeficiency Virus Type 2 (HIV-II), a Hepatitis A Virus (HAV), a Hepatitis C Virus (HCV), a Human T-Lymphotropic Virus Type 1 (HTLV-I), and a Human T-Lymphotropic Virus Type 2 (HTLV-II). The human DNA virus comprises a Hepatitis B Virus (HBV),a Parcovirus B19, an Epstein-Barr virus (EBV), a Human Cytomegalovirus (HCMV), a Human Adenovirus (HAdV), a Human Herpesvirus 6 (HHV-6), a Human Herpesvirus 7 (HHV-7), a Human Herpesvirus 8 (HHV-8), a Human Papillomavirus Type 16 (HPV-16), a Human Papillomavirus Type 18 (HPV-18), a BK virus and a JC virus. The bovine DNA virus comprises a Bovine Polyomavirus (BPyV). The porcine DNA virus comprises a Porcine Circovirus Type 1 (PCV-1), a Porcine Circovirus Type 2 (PCV-2) and a Porcine parvovirus (PPV). In practical applications, the virus species detected by the method for virus detection are not limited to those mentioned above, and the number of virus species can be increased or decreased according to user requirements or experimental design. Moreover, the origins of the virus should not be restricted to the aforementioned species, and may also comprise plants, animals or other biological species.

In Step S1, the solid-phase carrier can be a biochip, such as a protein chip or a gene chip. In Step S2, the sample can be a blood sample (e.g. plasma, serum, whole blood, etc.), a respiratory sample (e.g. saliva, nasal secretion, etc.), and organic sample (e.g. tissue section, biopsy sample, etc.). Inpractice, sample selection can be adjusted based on a specific user's requirements. In this specific embodiment, in Step S3, the amplification reaction temperature is below 70°C, and the amplification primer length is between 14 mer and 30 mer. The amplification primer concentration is between 0.1µM and 1µM, and the number of amplification reaction cycles is less than 40. In practice, however, it is not limited to this. In addition to the parameters mentioned above, the amplification reaction condition can also be selected based on users'requirements and experimental designs. Moreover, the parameter ranges and the optimized parameter valuesfor each amplification reaction condition can be adjusted and designed according to the equipment utilized by the user and the experimental requirements. In this specific embodiment, the method for virus detection detects a plurality of viruses originating from different species at one time by coating nucleic acid primer sequences corresponding to viruses originating from different species on the solid-phaseand adjusting and configuring the amplification reaction conditions.

In practice, an amplification reaction condition corresponding to each of the viruses can be determined through multiple trial-and-error tests to identify their corresponding parameter ranges and optimized parameter values. For example, it is necessary to design amplification reaction conditions that are applicable to each of the viruses when the method for virus detection aims to detect several human viruses at one time. The amplification reaction conditions comprise an amplification reaction temperature, an amplification primer length, an amplification primer concentration and an amplification reaction cycle. Wherein, each of the viruses comprises at least four of the aforementioned parameter ranges, and the optimized parameter values can be obtained by permuting and combining these parameter ranges. If the user aims to detect viruses originating from different species, more parameter ranges can be added for permutation and combination. In this process, the number of trial-and-error tests also increases. However, trial-and-error tests require a large amount of time and resources, and they often fail to find efficient solutions, especially under conditions involving complex problems or multiple attempt. Furthermore, repeated trial-and-error tests may consume a large amount of materials, human resources and energy, resulting in wasted costs, low efficiency, and a lack of systematic structure. Therefore, the following embodiment further describes how to efficiently identify the parameter range and optimize the parameter values of the amplification reaction conditions.

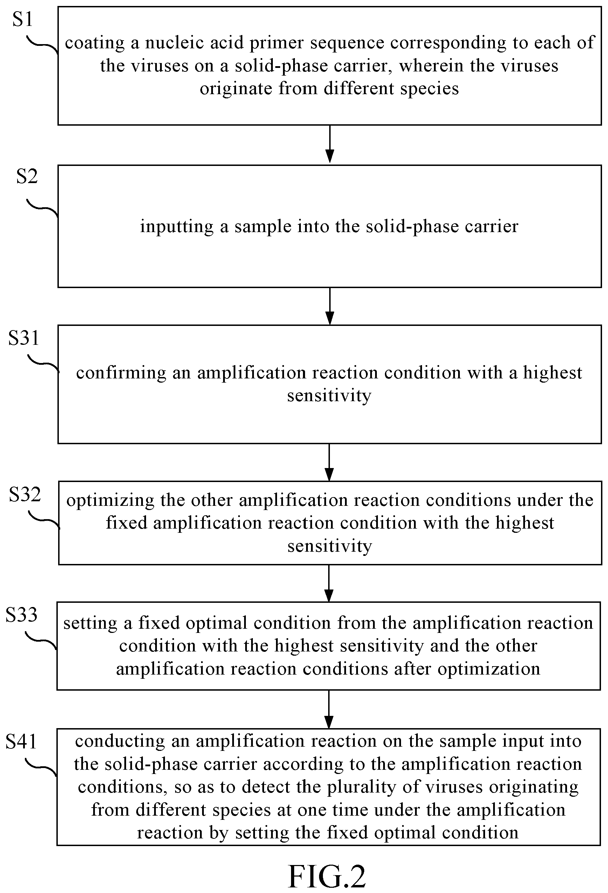

Please refer to FIG. 2. FIG. 2 is a flowchart illustrating the steps of the method for virus detection according to an embodiment of the present invention. As shown in FIG. 2. The difference between the aforementioned embodiment and this specific embodiment is that the step of setting amplification reaction conditions within the method for virus detection in this specific embodiment can further comprise Step S31, Step S32, Step S33 and Step S41.The aforementioned steps following the Step 2 can be conducted sequentially -Step S31: confirming an amplification reaction condition with a highest sensitivity; Step S32: optimizing the other amplification reaction conditions under the fixed amplification reaction condition with the highest sensitivity; Step S33: setting a fixed optimal condition from the amplification reaction condition with the highest sensitivity and the other amplification reaction conditions after optimization; and Step S41: conducting an amplification reaction on the sample input into the solid-phase carrier according to the amplification reaction conditions, so as to detect the plurality of viruses originating from different species at one time under the amplification reaction by setting the fixed optimal condition. In this specific embodiment, it is necessary to set the plurality of the amplification reaction conditions before conducting an amplification reaction. When adjusting one of the parameters within the amplification reaction conditions results in a statistical significance change in the amplification reaction result, the amplification reaction conditions can be considered as an amplification reaction condition with the highest sensitivity at that time.

For example, in the process of the amplification reaction, parameters such as amplification reaction temperature, amplification primer length and amplification reaction cycles can be adjusted respectively, resulting in variations in amplification reaction results. The parameter of the amplification reaction temperature can be fixed to further adjust the other amplification reaction conditions to identify their corresponding optimized parameter values when adjusting the amplification reaction temperature can lead to the statistical significance in the amplification reaction result that is determined in the process of the amplification reaction. Wherein, the other amplification reaction conditions comprise the amplification primer length, the amplification primer concentration and the amplification reaction cycles. Then, the fixed parameter value of the amplification reaction temperature and the optimized parameter values of the other amplification reaction conditions can be configured to detect the plurality of viruses originating from different species at one time under the amplification reaction. In this specific embodiment, the method for virus detection can replace prior art of repeated trial-and-error tests with confirming and fixing the amplification reaction condition with the highest sensitivity and optimizing the other amplification reaction conditions to reduce costly waste and promote the efficiency of the virus detection. It should be noted that the other steps in this specific embodiment and the corresponding steps in the abovementioned embodiment are roughly the same. Therefore, it will not be elaborated further.

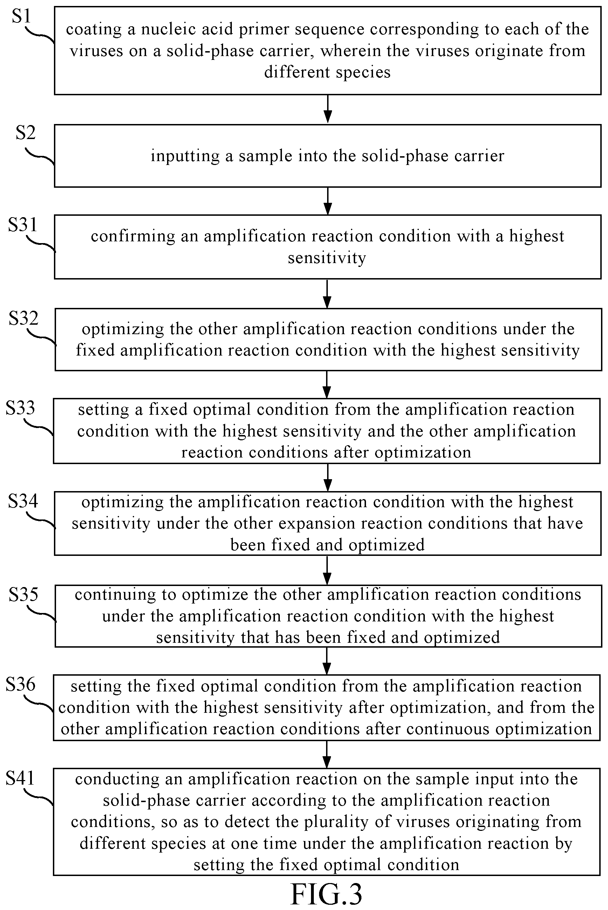

Furthermore, the step of setting amplification reaction conditions can comprise other forms in the method for the virus detection in the aforementioned embodiment. Please refer to FIG. 3.FIG. 3 is a flowchart illustrating the steps of the method for virus detection according to an embodiment of the present invention. As shown in FIG. 3, in this specific embodiment, the step of setting amplification reaction conditions can further comprise Step S34, Step S35 and Step S36. The above step follows Step S33. (Step S34: optimizing the amplification reaction condition with the highest sensitivity under the other expansion reaction conditions that have been fixed and optimized; Step S35: continuing to optimize the other amplification reaction conditions under the amplification reaction condition with the highest sensitivity that has been fixed and optimized; and Step S36: setting the fixed optimal condition from the amplification reaction condition with the highest sensitivity after optimization, and from the other amplification reaction conditions after continuous optimization.) In detail, when finding the other amplification reaction conditions, comprising the amplification primer length, the amplification primer concentration and the amplification reaction cycles, the fixed parameter value of the amplification reaction temperature can also be optimized correspondingly using the same method. Then, fix the optimized parameter values of the amplification reaction temperature and continue to optimize the other amplification reaction conditions. Furthermore, the invention can detect a plurality of viruses at one time under the amplification reactionby fixing the optimized parameter value of the amplification reaction temperature and the continuously optimized parameter values of the other amplification reaction conditions. The specific embodiment can improve the virus detection accuracy and reduce the likelihood of false positives and false negatives by further optimizing the amplification reaction condition with the highest sensitivity and continuously optimizing the other amplification reaction conditions. It should be noted that the other steps in this specific embodiment and the corresponding steps in the abovementioned embodiment are roughly the same. Therefore, it will not be elaborated further.

In summary, the present invention provides the method for virus detection. Compared to the conventional technology which only detects the virus originating from the same species, the invention can detect the plurality of viruses originating from different species at one time by coating a nucleic acid primer sequence corresponding to each of the viruses on a solid-phase carrier, and adjusting and designing an amplification reaction condition. In addition, compared to the conventional technology that requires costly and inefficient trial-and-error testing to adjust reaction conditions, the invention replaces the traditional trial-and-error test by identifying an amplification reaction condition with the highest sensitivity, thereby reducing cost and improving efficiency for virus detection. Moreover, compared to the insufficient precision of the conventional technology, the invention further increases the accuracy of the virus detection and reduces the incidence of false-negative or false-positive outcomes in viral diagnostics by optimizing the amplification reaction condition with the highest sensitivity and continuously optimizing the other amplification reaction conditions.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A method for virus detection, applied to detect a plurality of viruses at one time, and the method comprising the following steps of:
coating a nucleic acid primer sequence corresponding to each of the viruses on a solid-phase carrier, wherein the viruses originate from different species;
inputting a sample into the solid-phase carrier;
setting a plurality of amplification reaction conditions, wherein the amplification reaction conditions comprise an amplification reaction temperature, an amplification primer length, an amplification primer concentration and a number of amplification reaction cycles; and
conducting an amplification reaction on the sample input into the solid-phase carrier according to the amplification reaction conditions, so as to detect the plurality of viruses originating from different speciesat one time under the amplification reaction.

2. The method for virus detection of claim 1, wherein the amplification reaction temperature is below 70°C, the amplification primer length is between 14 mer and 30 mer, the amplification primer concentration is between 0.1 µM and 1 µM, and the number of amplification reaction cycles is less than 40.

3. The method for virus detection of claim 1, wherein the step of setting the plurality of amplification reaction conditions further comprises the following steps of:
Confirming an amplification reaction condition with a highest sensitivity;
Optimizing the other amplification reaction conditions under the fixed amplification reaction condition with the highest sensitivity; and
setting a fixed optimal condition from the amplification reaction condition with the highest sensitivity and the other amplification reaction conditions after optimization;
wherein, the step of conducting the amplification reaction on the sample input into the solid-phase carrier according to the amplification reaction conditions is conducting the amplification reaction by setting the fixed optimal condition.

4. The method for virus detection of claim 3,wherein the step of setting the plurality of amplification reaction conditions further comprises the following steps of:
optimizing the amplification reaction condition with the highest sensitivity under the other expansion reaction conditions that have been fixed and optimized;
continuing to optimize the other amplification reaction conditions under the amplification reaction condition with the highest sensitivity that has been fixed and optimized; and
setting the fixed optimal condition from the amplification reaction condition with the highest sensitivity after optimization, and from the other amplification reaction conditions after continuous optimization.

5. The method for virus detection of claim 1, further comprising a nucleic acid purification reaction, a reverse transcription reaction, a polymerase chain reaction, and an amplification reaction analysis.

6. The method for virus detection of claim 1, wherein the viruses comprise a human RNA virus, a human DNA virus, a bovine DNA virus and a porcine DNA virus.

7. The method for virus detection of claim 6, wherein the human RNA virus comprises a Human Immunodeficiency Virus Type 1 (HIV-I), a Human Immunodeficiency Virus Type 2 (HIV-II), a Hepatitis A Virus (HAV), a Hepatitis C Virus (HCV), a Human T-Lymphotropic Virus Type 1 (HTLV-I), and a Human T-Lymphotropic Virus Type 2 (HTLV-II).

8. The method for virus detection of claim 6, wherein the human DNA virus comprises a Hepatitis B Virus (HBV), a ParcovirusB19, an Epstein-Barr virus (EBV), a Human Cytomegalovirus (HCMV), a Human Adenovirus (HAdV), a Human Herpesvirus 6 (HHV-6), a Human Herpesvirus 7 (HHV-7), a Human Herpesvirus 8 (HHV-8), a Human Papillomavirus Type 16 (HPV-16), a Human Papillomavirus Type 18 (HPV-18), a BK virus and a JC virus.

9. The method for virus detection of claim 6, wherein the bovine DNA virus comprises a Bovine Polyomavirus (BPyV).

10. The method for virus detection of claim 6, wherein the porcine DNA virus comprises a Porcine Circovirus Type 1 (PCV-1), a Porcine Circovirus Type 2 (PCV-2) and a Porcine parvovirus (PPV).
